# FASCICULE DE BREVET EUROPEEN

(11) **EP 0 515 649 B1**
(45) Date de publication et mention de la délivrance du brevet: **08.11.1995**
(21) Numéro de dépôt: 92902079.0
(22) Date de dépôt: 13.12.1991
(51) Int. Cl.: H04N 5/225, H04N 9/04, A61B 1/04

(54) **ROUE DE FILTRES DE COULEUR POUR ENDOSCOPES ET TRAITEMENT DES SIGNAUX DE COULEUR GENERES**
FARBFILTERRAD FÜR ENDOSKOPE UN VERARBEITUNG DER ERZEUGTEN FARBSIGNALE
COLOUR FILTER WHEEL FOR ENDOSCOPES AND PROCESSING OF GENERATED COLOUR SIGNALS

(30) Priorité: 13.12.1990 FR 9015608
(43) Date de publication de la demande: 02.12.1992
(73) Titulaire: FORT FIBRES OPTIQUES RECHERCHE ET TECHNOLOGIE, F-91415 Dourdan Cédex (FR)
(72) Inventeur: FORT, François, F-75007 Paris (FR)
(74) Mandataire: Doireau, Marc
(86) Numéro de dépôt international: FR9101006
(87) Numéro de publication internationale: WO9210907

(56) Documents cités:
- US-A- 4 329 709
- US-A- 4 774 565
- US-A- 4 998 973
- PATENT ABSTRACTS OF JAPAN vol. 8, no. 17 (E-223)(1454) 25 Janvier 1984 & JP-A-58 179 083
- PATENT ABSTRACTS OF JAPAN vol. 12, no. 395 (E-671)20 Octobre 1988 & JP-A-63 136 787
- PATENT ABSTRACTS OF JAPAN vol. 13, no. 12 (P-812)12 Janvier 1989 & JP-A-63 220 215

## Description

La présente invention est relative à un procédé de captation et traitement d'images pour vidéo-endoscope couleur et à un vidéo-endoscope de mise en oeuvre de ce procédé.

Le Brevet FR-A-2 438 999 décrit un endoscope destiné au traitement d'informations de couleur et à la formation d'un signal de lecture, notamment compatible avec un appareil de télévision, (a priori quelconque et au format normalisé), c'est-à-dire destiné à former des images en couleurs d'un champ d'observation (à savoir, d'objets situés dans ce champ).

Ce Brevet français utilise un système d'éclairage basé sur les enseignements du Brevet US-A-4 074 306, dans lequel les informations de l'image observée dans le champ balayé par la tête d'observation de l'endoscope sont séparées en trois couleurs primaires, notamment Rouge, Verte, Bleue (RVB). L'image originale en couleurs est ensuite recréée par combinaison des images partielles en ces trois couleurs primaires RVB. Or, ces trois images RVB sont formées par filtrage mécanique de la lumière d'éclairage de la cavité à explorer (à l'aide de la tête d'observation de l'endoscope) en utilisant une source lumineuse unique associée à un disque rotatif de filtrage RVB de la lumière émise par celle-ci. En variante, les trois images RVB peuvent être aussi formées par séparation d'une image réfléchie du champ d'observation à l'aide d'une série de miroirs dichroïques.

Le Brevet français FR-A-2 438 999 diffère donc du Brevet américain US-A-4 074 306 essentiellement en ce que les trois images lumineuses séparées, contenant chacune des données correspondant aux couleurs primaires individuelles RVB, sont créées par commande successive d'une série de lampes ayant des courts temps de réponse.

Ce Brevet français, en outre, utilise une source de lumière individuelle pour chacune des couleurs primaires (RVB) utilisées dans le système vidéo adopté, cette lumière étant transmise au champ d'observation exploré par la tête de l'endoscope par l'intermédiaire d'un faisceau de fibres optiques. De plus, on forme successivement chacune des images de couleurs primaires RVB sur un dispositif de formation d'images à semi-conducteur, dont les signaux de sortie sont couplés électriquement à une mémoire destinée au stockage individuel de chacun des signaux de lecture de couleurs, ces signaux de couleurs enregistrés dans les mémoires précitées étant périodiquement transmis à un circuit de traitement d'images destiné à disposer l'information correspondante en un format compatible avec le format d'un récepteur couleur.

Toutefois, l'image ainsi reconstituée par voie analogique du champ visé ne présente pas les degrés de brillance et de contraste souhaitables dans ce genre d'application, où il est impératif de bien mettre en évidence les détails du champ d'observation contenant des informations optiques importantes.

L'abrégé publié le 12 Janvier 1989 dans la revue "Patent Abstracts of Japan" vol. 13, n° 12 (P-812) décrit un vidéo-endoscope couleur dans lequel un objet est éclairé à partir d'une source lumineuse avec interposition d'un filtre rotatif comprenant trois filtres de couleur R, Y et B disposés suivant trois secteurs, et on forme chacune des images sur un dispositif de formation d'images à semi-conducteur.

Cependant, l'image reconstituée ne présente pas les degrés de brillance et de contraste souhaitables pour ce genre d'application.

La présente invention s'est donc donné pour but d'éliminer les inconvénients de la technique antérieure, de manière à obtenir des images en couleurs du champ visé qui aient une brillance et un contraste meilleurs que ceux obtenus dans la technique antérieure, ce qui entraîne, comme avantage supplémentaire, l'augmentation d'au moins 30 % de la distance d'observation (de la tête de lecture du vidéo-endoscope par rapport au champ d'observation).

A cet effet, l'Invention propose un vidéo-endoscope couleur destiné à fournir des informations de couleurs d'un champ d'observation d'une cavité à explorer, ces informations étant sous une forme telle qu'elles puissent être affichées sur un récepteur vidéo de format standard par exemple, comprenant :
- une source d'éclairage unique,
- un faisceau de fibres optiques destiné à transmettre la lumière de cette source vers le champ d'observation,
- un dispositif de filtrage périodique de la lumière émise par la source précitée, comportant une première, une deuxième et une troisième zone filtrante permettant d'éclairer périodiquement, par l'intermédiaire du faisceau précité, le champ d'observation en trois couleurs primaires (RVB), respectivement, correspondant au passage de la lumière par chacune des trois zones filtrantes précitées, et une quatrième zone filtrante qui est transparente et traversée également par la lumière,
- un capteur photosensible destiné à capter une image d'objets situés dans le champ d'observation éclairé au travers du dispositif de filtrage,
- un convertisseur analogique/numérique pour convertir en numérique les informations périodiques issues du capteur et représentatives d'images en couleurs (R, V, B) et d'une image noir et blanc (N-Bl), les informations numériques étant stockées dans quatre mémoires correspondant respectivement aux informations de couleurs (R, V, B) et en noir et blanc (N-Bl),
- un dispositif de nuancement en couleurs combinant, de manière sélective, les informations de l'image en noir et blanc (N-Bl) avec les informations de couleurs,
- un convertisseur numérique/analogique pour convertir en analogique les signaux numériques en sortie du dispositif de nuancement,
- et un codeur, formant interface avec un appareil de visualisation, pour reconstituer une image en couleurs,

le dispositif de nuancement en couleurs comprenant au moins trois circuits d'addition pour additionner respectivement les signaux de couleurs verte et bleue stockés dans les mémoires, les signaux de couleurs rouge et bleue stockés dans les mémoires et les signaux de couleur rouge et verte stockés dans les mémoires, et comprenant également au moins trois circuits de soustraction reliés en entrée aux trois circuits d'addition respectivement, et à la mémoire où sont stockés les signaux en noir et blanc, les sorties des circuits de soustraction étant reliées au convertisseur numérique/analogique.

Selon un second mode de réalisation, le dispositif de nuancement couleurs comprend trois circuits d'addition pour additionner les signaux en noir et blanc stockés dans la mémoire associée avec les signaux en couleurs respectivement stockés dans les autres mémoires.

L'Invention a également pour objet un procédé de captation et de traitement d'images en couleurs à l'aide d'un vidéo-endoscope tel que défini par l'une des Revendications précédentes, comprenant les étapes consistant :
- à éclairer un champ d'observation par une source de lumière et un faisceau de fibres optiques de transmission de la lumière,
- à filtrer périodiquement la lumière émise par la source au moyen de trois zones de filtrage pour éclairer le champ d'observation suivant les trois couleurs primaires (R, V, B), et d'une quatrième zone de filtrage transparente laissant passer la lumière,
- à capter périodiquement au moyen d'un capteur photosensible les images réfléchies par le champ d'observation et correspondant respectivement à des images de couleurs rouge, verte, et bleue, et une image en noir et blanc,
- à convertir en numérique les informations recueillies par le capteur et à les stocker dans quatre mémoires correspondant aux signaux des images de couleurs rouge, verte et bleue, et aux signaux de l'image en noir et blanc,
- et à traiter de façon sélective les signaux stockés dans les mémoires sur la base des signaux en noir et blanc pour nuancer en couleurs une image reconstituée à partir des signaux ainsi traités,

l'étape de traitement sélectif des signaux stockés dans les mémoires consistant à additionner respectivement les signaux de couleurs verte et bleue, les signaux de couleurs rouge et bleue, et les signaux de couleurs rouge et verte, et à soustraire ensuite de chacun des signaux obtenus les signaux en noir et blanc pour reconstituer une image en couleurs de qualité.

Selon une autre caractéristique de l'Invention, le procédé consiste à traiter les signaux stockés dans les mémoires en effectuant des opérations d'addition et il consiste notamment à additionner les signaux en noir et blanc aux signaux de couleurs rouge, verte et bleue, respectivement, et à combiner les signaux ainsi additionnés pour reconstituer une image en couleurs, les opérations d'addition étant avantageusement des opérations pondérées.

Il est ainsi possible d'obtenir une meilleure qualité d'image, grâce aux informations complémentaires, notamment de sensibilité, contenues dans l'image en noir et blanc.

L'invention sera mieux comprise à l'aide du complément de description qui va suivre, fait en référence aux dessins annexés, dans lesquels :
- la figure 1 illustre schématiquement le principe de fonctionnement du vidéo-endoscope conforme à l'invention,
- et la figure 2 illustre schématiquement une variante des circuits de traitement utilisés pour reconstituer une image en couleurs.

La référence numérique 10 représente une lampe à arc disposée au centre d'un radiateur (non représenté), du type à étoile et destiné à coopérer avec un ventilateur (également non représenté), pour provoquer une ventilation forcée de refroidissement de la lampe (ayant une puissance d'environ 300 W dans le mode de réalisation illustré) : étant donné que l'arc électrique est amorcé à l'aide d'une tension très élevée (de l'ordre de 5 000 V), des mesures appropriées de sécurité d'emploi -à la portée de l'homme du métier- doivent être prévues.

Un dispositif anti-calorique 20 fait suite à la lampe 10. Ce dispositif comporte deux filtres anti-caloriques 21 et 22, qui sont écartés de façon appropriée l'un par rapport à l'autre et permettent d'assurer la nécessaire protection thermique par suppression de la radiation infrarouge (IR) de la lumière émise par la lampe 10.

Un diaphragme (non représenté) est généralement utilisé pour régler l'intensité lumineuse, et ce sous contrôle électronique, suivant des dispositions connues des techniciens en la matière.

Une lentille (un condenseur) 30 permet de focaliser le faisceau lumineux en provenance de la lampe 10 dans l'axe d'un faisceau de fibres optiques 40.

Entre le dispositif anti-calorique 20 et ce faisceau 40 est disposé un dispositif 50 de filtrage de la lumière émise par la lampe 10.

Ce dispositif de filtrage comporte un disque 51 tournant sous l'action d'un moteur 52 (la transmission du mouvement se faisant par une courroie 57, qui est du type cranté) et comportant quatre zones de filtrage. Les trois zones de filtrage 53, 54, 55 (dont la présence est classique dans ce genre de dispositif), visent à éclairer périodiquement, en trois couleurs primaires ou fondamentales, notamment Rouge, Verte et Bleue (RVB), respectivement, le champ d'observation (non représenté) à l'intérieur d'une cavité à explorer. La quatrième zone 56 est transparente, c'est-à-dire qu'elle laisse passer telle quelle (donc sans filtrage) la lumière émise par la lampe 10.

Cela revient à dire que le disque de filtrage 51 est divisé en quatre cadrans, dont chacun est occupé par une des zones précitées 53 à 56. De façon plus précise, dans le mode de réalisation objet de la présente description, les zones opaques du disque rotatif 51 sont définies par des secteurs d'environ 35°, alors que les zones filtrantes 53 à 55 et la zone transparente 56 sont définies par des secteurs d'environ 55°, les zones filtrantes précitées étant constituées par des filtres RVB, respectivement, qui sont de type connu des techniciens en la matière.

Bien entendu, entre deux zones contiguës existe une zone "d'ombre", c'est-à-dire une zone opaque à la lumière émise par la source 10. Or, c'est pendant le temps nécessaire pour passer d'une zone du disque tournant 51 à la zone consécutive que s'effectue le traitement des informations optiques dans chacune des trois couleurs primaires correspondant à chacun des éclairages RVB ou Bl (c'est-à-dire en lumière blanche Bl, à savoir sans filtrage) du champ d'observation. Les images RVB et en Noir et Blanc (N-Bl) du champ d'observation ainsi obtenues sont captées par un capteur photo-sensible 60, qui est constitué de préférence par un dispositif à semiconducteur, tel qu'une caméra CCD, et qui est disposé à côté de l'extrémité distale 41 du faisceau de fibres optiques 40, dans la tête d'observation précitée du vidéo-endoscope 100 (en pratique, les fibres du faisceau 30, dans la tête d'observation du vidéo-endoscope, sont disposées sous forme de croissant de lune, le reste de la zone circulaire occupée par la tête d'observation étant occupé par la caméra CCD).

En ce qui concerne la tête d'observation, celle-ci est avantageusement du type constitué par des anneaux articulés entre eux et susceptibles d'être commandés par un câble, à l'aide d'une poignée de manoeuvre, ces dispositions étant connues des techniciens en la matière.

Les câbles optiques et électriques sont de préférence réunis en un câble unique en Y.

Les informations optiques captées par la caméra CCD sont converties en numérique par un convertisseur analogique/numérique 91, et ces informations numériques sont ensuite stockées dans quatre mémoires 81 à 84 correspondant aux signaux en noir et blanc, respectivement. La mémorisation de ces informations numériques est effectuée au travers d'un dispositif de commutation 71 piloté par un dispositif de commande 70 qui assure la synchronisation de cette opération de mémorisation avec l'opération de filtrage effectuée par le disque de filtrage tournant 51.

Les signaux enregistrés dans les mémoires 81 à 84 sont ensuite traités d'une manière sélective, sachant que les signaux correspondant à l'image en noir et blanc N-Bl contiennent des informations complémentaires, notamment au niveau de la sensibilité, par rapport à celles contenues dans l'ensemble des signaux correspondant aux images de couleurs rouge, verte et bleue. On élabore donc des combinaisons de signaux à partir de ceux enregistrés dans les mémoires 81 à 84, au moyen de circuits de traitement qui exécutent des fonctions d'addition et de soustraction par exemple. Ces circuits constituent globalement un dispositif de nuancement de couleurs en vue de reconstituer une image finale en couleurs dont la qualité est nettement améliorée par rapport à une image reconstituée à partir de la seule combinaison des trois images en couleurs.

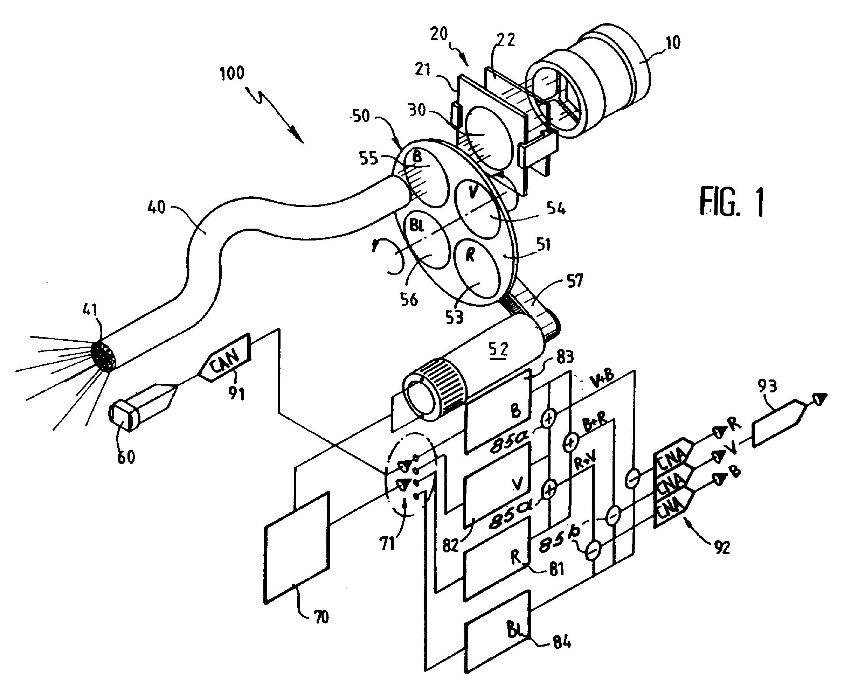

Selon un premier mode de réalisation de ce dispositif de nuancement, tel qu'illustré à la figure 1, on combine les signaux représentatifs de l'image de couleur verte stockés dans la mémoire 82, dont l'amplitude correspond à l'intensité V de la couleur verte, et les signaux représentatifs de l'image de la couleur bleue stockés dans la mémoire 83, dont l'amplitude correspond à l'intensité B de la couleur bleue. Ces signaux sont combinés dans un circuit de traitement 85a, qui effectue globalement une opération d'addition, et ils ont alors une amplitude qui correspond à l'intensité de l'image d'origine moins l'intensité R de l'image de couleur rouge. Ensuite, les signaux combinés par le circuit de traitement 85a et les signaux en noir et blanc stockés dans la mémoire 84, sont traités par un circuit de traitement 85b, qui effectue globalement une opération de soustraction, pour obtenir ainsi en sortie des signaux dont l'amplitude correspond à une intensité R d'une image de couleur rouge qui est améliorée par rapport à l'image de couleur rouge d'origine.

On procède de la même manière en combinant les signaux représentatifs des images de couleurs bleue et rouge dans un circuit de traitement 85a, et les signaux résultants sont combinés avec les signaux en noir et blanc dans un circuit de traitement 85b, pour obtenir en sortie des signaux dont l'amplitude correspond à une intensité V d'une image de couleur verte qui est améliorée par rapport à l'image de couleur verte d'origine. Enfin, on combine les signaux des images de couleurs rouge et verte dans un circuit de traitement 85a, et les signaux résultant sont combinés avec les signaux en noir et blanc dans un circuit de traitement 85b, pour obtenir en sortie des signaux dont l'amplitude correspond à une intensité B d'une image de couleur bleue qui est améliorée par rapport à l'image de couleur bleue d'origine.

Les signaux de sortie des circuits de traitement 85b sont ensuite convertis en signaux analogiques par un convertisseur numérique/analogique 92, et une image en couleurs est reconstituée à l'aide d'un codeur 93 compatible avec un appareil de télévision au format normalisé par exemple, choisi notamment parmi les standards vidéo, tels que NTSC, PAL ou SECAM.

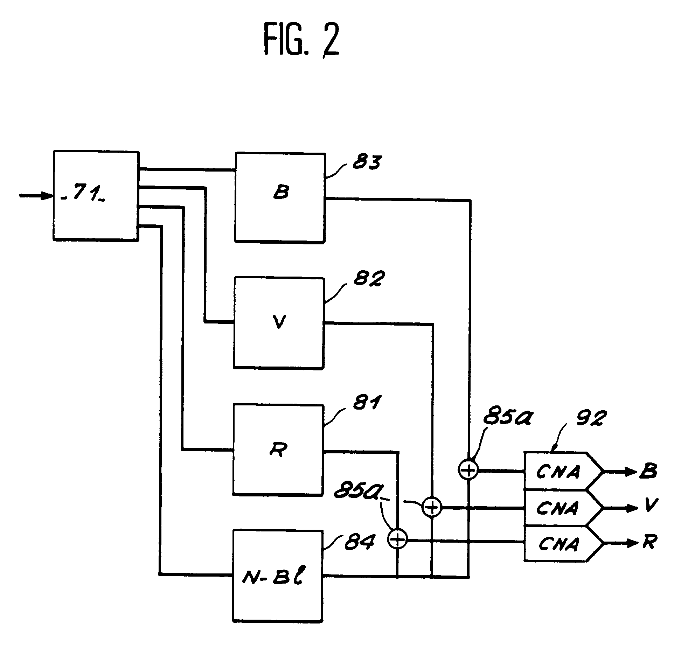

En variante, comme illustré à la figure 2, une autre possibilité de traitement des signaux stockés dans les mémoires 81 à 84, consiste à combiner dans trois circuits de traitement 85a, du type précité, les signaux en noir et blanc stockés dans la mémoire 84 avec les signaux respectivement stockés dans les mémoires 81 à 83. Ainsi, en sortie de ces trois circuits de traitement 85a, on obtient des signaux correspondant à trois images en noir et blanc colorées respectivement en rouge, en vert et en bleu, chacune de ces images ayant une qualité améliorée par rapport à l'image correspondante de couleur rouge, verte ou bleue d'origine. Ensuite, on reconstitue une image en couleurs à partir des signaux de ces trois images, au moyen du convertisseur numérique/analogique 92 et du codeur 93, comme précédemment.

D'une manière avantageuse, on peut associer aux circuits de traitement 85a et 85b des fonctions de pondération pour jouer sur les intensités des couleurs rouge, verte et bleue, et nuancer ainsi les couleurs de l'image reconstituée afin d'en améliorer la qualité.

Il va de soi que les applications du vidéo-endoscope conforme à l'invention peuvent être non seulement médicales, mais aussi industrielles.

Ainsi que cela ressort de ce qui précède, l'invention ne se limite nullement à ceux de ses modes de mise en oeuvre de réalisation et d'application qui viennent d'être décrits de façon plus explicite ; elle en embrasse, au contraire, toutes les variantes qui peuvent venir à l'esprit du technicien en la matière, sans s'écarter du cadre, ni de la portée, de la présente invention.

## Revendications

1. Vidéo-endoscope couleur (100) destiné à fournir des informations de couleurs d'un champ d'observation d'une cavité à explorer, ces informations étant sous une forme telle qu'elles puissent être affichées sur un récepteur vidéo de format standard par exemple, comprenant :
- une source d'éclairage unique (10),
- un faisceau de fibres optiques (40) destiné à transmettre la lumière de cette source (10) vers le champ d'observation,
- un dispositif de filtrage périodique (50) de la lumière émise par la source précitée (10), comportant une première, une deuxième et une troisième zone filtrante (53,54,55) permettant d'éclairer périodiquement, par l'intermédiaire du faisceau précité (40), le champ d'observation en trois couleurs primaires (RVB), respectivement, correspondant au passage de la lumière par chacune des trois zones filtrantes précitées (53,54,55), et une quatrième zone filtrante (56) qui est transparente et traversée également par la lumière,
- un capteur photosensible (60) destiné à capter une image d'objets situés dans le champ d'observation éclairé au travers du dispositif de filtrage (50),
- un convertisseur analogique/numérique (91) pour convertir en numérique les informations périodiques issues du capteur (60) et représentatives d'images en couleurs (R, V, B) et d'une image noir et blanc (N-Bl), les informations numériques étant stockées dans quatre mémoires (81 à 84) correspondant respectivement aux informations de couleurs (R, V, B) et en noir et blanc (N-Bl),
- un dispositif de nuancement en couleurs (85a,85b) combinant, de manière sélective, les informations de l'image en noir et blanc (N-Bl) avec les informations de couleurs,
- un convertisseur numérique/analogique (92) pour convertir en analogique les signaux numériques en sortie du dispositif de nuancement (85a,85b), et
- un codeur (93), formant interface avec un appareil de visualisation, pour reconstituer une image en couleurs,
le dispositif de nuancement en couleurs comprenant au moins trois circuits d'addition (85a) pour additionner respectivement les signaux de couleurs verte et bleue stockés dans les mémoires (82,83), les signaux de couleurs rouge et bleue stockés dans les mémoires (81,83) et les signaux de couleur rouge et verte stockés dans les mémoires (81 et 82), et comprenant également au moins trois circuits de soustraction (85b) reliés en entrée aux trois circuits d'addition (85a) respectivement, et à la mémoire (84) où sont stockés les signaux en noir et blanc, les sorties des circuits de soustraction (85b) étant reliées au convertisseur numérique/analogique (92).

2. Vidéo-endoscope couleur selon la Revendication 1, caractérisé en ce qu'il comprend un dispositif de commutation (70,71) pour piloter le stockage des informations captées par le capteur (60) et converties en numérique par le convertisseur (91), en synchronisme avec la rotation du dispositif de filtrage (50).

3. Procédé de captation et de traitement d'images en couleurs à l'aide d'un vidéo-endoscope tel que défini par l'une des Revendications précédentes, comprenant les étapes consistant :
- à éclairer un champ d'observation par une source de lumière et un faisceau de fibres optiques de transmission de la lumière,
- à filtrer périodiquement la lumière émise par la source au moyen de trois zones de filtrage pour éclairer le champ d'observation suivant les trois couleurs primaires (R, V, B), et d'une quatrième zone de filtrage transparente laissant passer la lumière,
- à capter périodiquement au moyen d'un capteur photosensible les images réfléchies par le champ d'observation et correspondant respectivement à des images de couleurs rouge, verte, et bleue, et une image en noir et blanc,
- à convertir en numérique les informations recueillies par le capteur et à les stocker dans quatre mémoires correspondant aux signaux des images de couleurs rouge, verte et bleue, et aux signaux de l'image en noir et blanc, et
- à traiter de façon sélective les signaux stockés dans les mémoires sur la base des signaux en noir et blanc pour nuancer en couleurs une image reconstituée à partir des signaux ainsi traités,
l'étape de traitement sélectif des signaux stockés dans les mémoires consistant à additionner respectivement les signaux de couleurs verte et bleue, les signaux de couleurs rouge et bleue, et les signaux de couleurs rouge et verte, et à soustraire ensuite de chacun des signaux obtenus les signaux en noir et blanc pour reconstituer une image en couleurs de qualité.

## Patentansprüche

1. Farbvideo-Endoskop (100), das dazu bestimmt ist, Farbinformationen von einem Beobachtungsfeld eines zu untersuchenden Hohlraums zu liefern, wobei diese Informationen in einer solchen Form sind, daß sie auf einem Video-Empfänger, z.B. von Standardformat, dargestellt werden können, umfassend:
- eine einzige bzw. einzelne Beleuchtungsquelle (10),
- ein Bündel von optischen Fasern (40), das dazu bestimmt ist, das Licht von dieser Quelle (10) nach dem Beobachtungsfeld zu übertragen,
- eine Einrichtung (50) zur periodischen Filterung des von der vorgenannten Quelle (10) emittierten Lichts, die eine erste, eine zweite und eine dritte filternde Zone (53, 54, 55) hat, welche es durch die Vermittlung des vorgenannten Bündels (40) gestatten, das Beobachtungsfeld in drei Primärfarben (RVB) jeweils entsprechend dem Durchgang des Lichts durch jede der drei vorgenannten filternden Zonen (53, 54, 55) zu beleuchten, und eine vierte filternde Zone (56), die transparent ist und ebenfalls von dem Licht durchquert wird,
- einen lichtempfindlichen Aufnehmer (60), der dazu bestimmt ist, ein Bild der Objekte, die sich in dem Beobachtungsfeld, das durch die Einrichtung (50) zur Filterung beleuchtet ist, aufzunehmen,
- einen Analog-zu-Digital-Umsetzer (91) zum Umwandeln der periodischen Informationen, die von dem Aufnehmer (60) hervorgegangen und für Farbbilder (R, V, B) und ein Schwarzweißbild (N-Bl) repräsentativ sind, in Digitalinformationen, wobei die digitalen Informationen in vier Speichern (81 bis 84), welche jeweils den Farbinformationen (R, V, B) und den Schwarzweißinformationen (N-Bl) entsprechen, gespeichert werden,
- eine Farbabstufungseinrichtung (85a, 85b), welche in selektiver Art und Weise die Informationen des Schwarzweißbilds (N-Bl) mit den Farbinformationen kombiniert,
- einen Digital-zu-Analog-Umsetzer (92) zum Umsetzen der Digitalsignale am Ausgang der Abstufungseinrichtung (85a, 85b) in Analogsignale, und
- einen eine Schnittstelle mit einer Sichtbarmachungseinrichtung bildenden Kodierer (93) zum Rekonstruieren eines Farbbilds,
wobei die Farbabstufungseinrichtung wenigstens drei Additionsschaltungen (85a) zum jeweiligen Addieren der in den Speichern (82, 83) gespeicherten Signale von grüner und blauer Farbe, der in den Speichern (81, 83) gespeicherten Signale von roter und blauer Farbe, und der in den Speichern (81 und 82) gespeicherten Signale von roter und grüner Farbe umfaßt, und ebenfalls wenigstens drei Subtraktionsschaltungen (85b) umfaßt, die am Eingang jeweils mit den drei Additionsschaltungen (85a) und mit dem Speicher (84), wo die Schwarzweißsignale gespeichert sind, verbunden sind, wobei die Ausgänge der Subtraktionsschaltungen (85b) mit dem Digital-zu-Analog-Umsetzer (92) verbunden sind.

2. Farbvideo-Endoskop gemäß Anspruch 1, dadurch **gekennzeichnet**, daß es eine Umschaltungseinrichtung (70, 71) zum Steuern der Speicherung der von dem Aufnehmer (60) aufgenommenen und von dem Umsetzer (91) in Digitalinformationen umgesetzten Informationen in Synchronismus mit der Drehung der Einrichtung (50) zur Filterung umfaßt.

3. Verfahren zur Aufnahme und zur Verarbeitung von Farbbildern mit Hilfe eines Video-Endoskops, wie es durch einen der vorhergehenden Ansprüche definiert ist, umfassend die Schritte, bestehend:
- im Beleuchten eines Beobachtungsfelds durch eine Lichtquelle und ein Bündel optischer Fasern für die Übertragung des Lichts,
- im periodischen Filtern des durch die Quelle emittierten Lichts mittels drei Filterungszonen für das Beleuchten des Beobachtungsfelds gemäß den drei Primärfarben (R, V, B), und eine vierte, transparente Zone der Filterung, welche das Licht durchgehen läßt,
- im periodischen Aufnehmen der von dem Beobachtungsfeld reflektierten und jeweils Bildern in den Farben Rot, Grün und Blau und einem Schwarzweißbild entsprechenden Bildern mittels eines lichtempfindlichen Aufnehmers,
- im Umwandeln der durch den Aufnehmer aufgefangenen Informationen in Digitalinformationen und im Speichern der Signale der Bilder von roter, grüner und blauer Farbe und der Signale des Schwarzweißbilds in vier entsprechenden Speichern, und
- im Verarbeiten der in den Speichern gespeicherten Signale in selektiver Art und Weise auf der Basis der Signale in Schwarz und Weiß zum Farbabstufen eines von den so behandelten Signalen rekonstruierten Bilds,
- wobei der Schritt der selektiven Verarbeitung der in den Speichern gespeicherten Signale im jeweiligen Addieren der Signale von grüner und blauer Farbe, der Signale von roter und blauer Farbe, und der Signale von roter und grüner Farbe, und danach im Subtrahieren der Schwarz- und Weißsignale von jedem der erhaltenen Signale zum Rekonstruieren eines Qualitätsfarbbilds.

## Claims

1. A color video-endoscope (100) for providing color information concerning a field of observation in a cavity to be explored, said information being in a form such as to enable it to be displayed on a standard format video receiver, for example, the video-endoscope comprising :
- a single lighting source (10) ;
- a bundle of optical fibers (40) for conveying light from said source (10) to the field of observation ;
- a periodic filtering device (50) for filtering the light emitted by the above-mentioned source (10), and including first, second, and third filter zones (53, 54, 55) enabling the field of observation to be periodically illuminated via the above-mentioned bundle (40) with three primary colors (RGB) respectively corresponding to light passing through each of the three above-mentioned filter zones (53, 54, 55), and a fourth filter zone (56) which is transparent and through which the light also passes ;
- a photosensitive sensor (60) designed to pick up an image of objects situated in the field of observation illuminated through the filter device (50) ;
- an analog-to-digital converter (91) for digitizing the periodic information from the sensor (60) and representative of color images (R, G, B) and a black-and-white image (B/W), with the digital information being stored in four memories (81 to 84) corresponding respectively to the color information (R, G, B) and to the black-and-white information (B/W) ;
- a color toning device (85a, 85b) for selectively combining the black-and-white image information (B/W) with the color information ;
- a digital-to-analog converter (92) for converting the digital signals output by the toning device (85a, 85b) into analog form ; and
- an encoder (93) forming an interface with a display apparatus for reconstituting a color image,
the color toning device comprising three adder circuits (85a) for adding together respectively the green and blue color signals stored in the memories (82, 83), the red and blue color signals stored in the memories (81, 83), and the red and green color signals stored in the memories (81, 82), and including also three subtractor circuits (85b) having their inputs connected to respective ones of the three adder circuits (85a) and to the memory (84) in which the black-and-white signals are stored, with the outputs from the subtractor circuits (85b) being connected to the digital-to-analog converter means (92).

2. A color video-endoscope according to claim 1, characterized in that it includes a switching device (70, 71) for controlling the storage of the information sent by the sensor (60) and converted by the converter (91) synchronously with rotation of the filtering device (50).

3. A method of sensing and processing color images by means of a video-endoscope as defined by any preceding claim, the method comprising the following steps :
- illuminating a field of observation by means of a light source and a bundle of optical fibers for transmitting light ;
- periodically filtering the light emitted by the source using three filter zones to illuminate the field of observation with three primary colors (R, G, B), and a fourth filter zone that is transparent and allows the light to pass ;
- using a photosensitive sensor to periodically sense the images reflected by the field of observation and corresponding respectively to red, green, and blue color images and to a black-and-white image ;
- converting the information picked up by the sensor into digital form and storing it in four memories corresponding to the red, green, and blue color image signals and to the black-and-white image signals ; and
- selectively processing the signals stored in the memories on the basis of the black-and-white signals to tone the colors of an image reconstituted from the signals processed in this way,
the selective processing step of the signals stored in the memories consisting in adding the green and blue colors signals, the red and blue color signals, and the red and green signals respectively, and then in subtracting from each resulting signals the black-and-white color signals in order to reconstitute a color image of good quality.
